# EUROPÄISCHE PATENTSCHRIFT

(11) **EP 1 432 469 B1**
(45) Veröffentlichungstag und Bekanntmachung des Hinweises auf die Patenterteilung: **06.08.2008**
(21) Anmeldenummer: 02760029.5
(22) Anmeldetag: 23.09.2002
(51) Int. Cl.: A61M 39/10, A61M 39/00

(54) **VORRICHTUNG ZUR KATHETERKONNEKTION**
CATHETER CONNECTION DEVICE
DISPOSITIF POUR LE RACCORD D'UN CATHETER

(30) Priorität: 05.10.2001 DE 10149051
(43) Veröffentlichungstag der Anmeldung: 30.06.2004
(73) Patentinhaber: Disetronic Licensing AG, 3401 Burgdorf (CH)
(72) Erfinder: DENOTH, Patrik, CH-1797 MÜNCHENWILER (CH); NIEDERHÄUSER, Sandro, CH-4914 ROGGWIL (CH); VALIQUER, Astrid, CH-3225 MÜNTCHEMIER (CH)
(74) Vertreter: Gassenhuber, Andreas
(86) Internationale Anmeldenummer: PCT/CH2002/000529
(87) Internationale Veröffentlichungsnummer: WO 2003/030985

(56) Entgegenhaltungen:
- WO-A-83/00812
- DE-U- 9 013 145
- US-A- 4 256 106
- US-A- 4 334 551

## Beschreibung

Die vorliegende Erfindung bezieht sich auf eine Vorrichtung zur Katheterkonnektion, insbesondere auf eine implantierbare Konnektion zur Verbindung mindestens zweier Katheter bzw. Katheterenden für einen intravenösen Zugang, sowie auf ein Verfahren zum Verbinden, mindestens zweier katheterenden mittels einer Katheterkonnektion.

Aus der WO 99/53981 ist eine Katheterverbindung für ein subkutanes Portsystem bekannt. Dabei wird ein Ende eines an einem Porfkörper anzubringenden Katheters auf einen Träger des Portkörpers aufgeschoben und mit einer Hülse wird das aufgeschobene Katheterstück aufgepresst und somit an dem Portkörper befestigt. Die Befestigung unmittelbar am Portkörper führt jedoch dazu, dass verschiedene Handgriffe am Portkörper selbst erforderlich sind, was insbesondere störend ist, wenn der Portkörper gerade implantiert ist und mit dem Katheter verbunden werden soll, da diese Handgriffe unmittelbar im Bereich des geöffneten Gewebes durchgeführt werden müssen.

Aus der US 4,256,106 sind zwei Konnektorteilstücke bekannt, wobei ein Katheter auf ein Teil aufgeschoben und mit einem Element in Position gehalten werden kann. Ein Konnektorelement ist unlösbar mit einem Rohr verbunden.

Eine ähnliche Struktur zur Verbindung eines Katheters mit einem Rohr mittels zweier Konnektorelemente ist auch aus der US 4,334,551 bekannt.

Das deutsche Gebrauchsmuster 90 13 145 offenbart eine medizinische Schlauchverbindungsvorrichtung mit einem ersten Anschlussstück, das einen Aufnahmekanal mit einer aufstoßbaren selbstschließenden elastischen Wand aufweist und einem an das erste Anschlussstück ankuppelbaren zweiten Anschlussstück, das eine das erste Anschlussstück übergreifende Fassung und einen die elastische Wand aufstoßenden hohlen Zapfen aufweist, und mit an den Anschlussstücken vorgesehenen ersten und zweiten Eingriffsteilen, die ein Auseinanderziehen der Anschlussstücke verhindern, wobei die ersten Eingriffsteile in Längsrichtung verlaufende Rastausnehmungen sind und die zweiten Eingriffsteile radial federnde Rastnasen sind, die bei verdrehungsfreier relativer Längsverschiebung beider Anschlussstücke in die Rastausnehmungen erst einfallen, nachdem der Zapfen die elastische Wand aufgestoßen hat.

Es ist eine Aufgabe der vorliegenden Erfindung, eine Katheterkonnektion zur Verfügung zu stellen, welche das Verbinden eines Katheters mit einem Portkörper vereinfacht.

Diese Aufgabe wird durch eine Katheterkonnektion wie in Anspruch 1 definiert, gelöst. Vorteilhafte Ausführungsformen ergeben sich aus den Unteransprüchen.

Gemäß der vorliegenden Erfindung wird eine Katheterkonnektion mit einem wie oben beschriebenen Träger und mindestens zwei wie oben beschriebenen Hülsen offenbart, womit eine Verbindung zwischen zwei Katheterenden hergestellt werden kann.

Es ist ein Träger zum Befestigen von mindestens zwei oder auch drei oder mehrerer Katheterenden bzw. Kathetern vorgesehen mit mindestens zwei Ansetzelementen, an welche die Katheterelemente angelegt, zum Beispiel aufgeschoben, werden können. Dabei sind die Ansetzelemente bevorzugt zylinderförmig und vorteilhaft innen hohl ausgestaltet, wobei der Innendurchmesser eines Ansatzelements vorteilhaft in etwa gleich groß ist wie der Innendurchmesser des Katheters, welcher konnektiert werden soll. Weiterhin sind mindestens zwei Verbindungsstrukturen vorgesehen, an welcher jeweils eine Hülse, welche bevorzugt vor dem Anlegen oder Aufschieben des Katheterelements auf das Ansetzelement auf den Katheter aufgeschoben wurde, an dem Träger befestigt werden kann. Dabei wird die auf den Katheter aufgeschobene Hülse nach dem Ansetzen des Katheters in Richtung des zu konnektierenden Katheterendes geschoben, bis die Hülse zur Verbindungsstruktur des Trägers gelangt, so dass die Hülse mit der Verbindungsstruktur verbunden werden kann und hierdurch eine feste Verbindung, bevorzugt eine kraftschlüssige, formschlüssige und/oder reibschlüssige Verbindung zum Beispiel durch Einklemmen des Katheterendes zwischen Träger und Hülse hergestellt werden kann.

Die Verbindungsstruktur, an welcher die Hülse befestigt werden kann, ist vorzugsweise ein Gewinde oder Gewindeabschnitt, so dass die Hülse auf den Träger aufgeschraubt werden kann. Besonders vorteilhaft ist im Bereich des Gewindes, zum Beispiel benachbart zu dem Gewinde, eine Struktur für eine Schnapp- oder Rastverbindung vorgesehen, wie zum Beispiel eine um den Träger umlaufende Rille oder abschnittsweise Vertiefung, in welche korrespondierende Vorsprünge oder Schnapphaken der Klemmhülse eingreifen können, um die Hülse zum Beispiel gegen unbeabsichtigtes Lösen zu sichern und vorteilhaft die Verbindung zwischen Träger und Klemmhülse über das Gewinde zu entlasten. Die Oberflächenstruktur für die Schnapp- oder Rastverbindung kann auch ohne ein Gewinde am Träger vorgesehen sein.

Ein Ansetzelement des Trägers, an welches das Katheterende angelegt wird, kann zum Beispiel eine umlaufend glatte, geriffelte oder strukturierte Oberfläche aufweisen, wobei eine strukturierte, zum Beispiel mehrere Vorsprünge oder kleine Erhebungen aufweisende Oberfläche vorteilhaft zum Anbringen oder Befestigen eines Katheterendes zum Beispiel durch Einklemmen in Verbindung mit einer Klemmhülse ist. Das Ansatzelement ist bevorzugt etwa röhrenförmig ausgebildet.

Es sind Hülsen vorgesehen, welche an je einem Katheterende angebracht werden können und bevorzugt einen Durchgang aufweisen, um auf den Katheter aufgeschoben werden zu können. Die Hülse ist vorzugsweise zumindest abschnittsweise konisch ausgebildet, d.h. der Außendurchmesser der Hülse verringert sich von der Hülsenseite, welche zur Verbindung mit dem Träger dient in Richtung auf das Hülsenende, welches im konnektierten Zustand von dem Träger wegweist. Eine solche konische Ausgestaltung einer Hülse ist vorteilhaft beim Bewegen einer Katheterkonnektion, welche von Gewebe umgeben ist, da aufgrund der konischen Ausgestaltung umliegendes Gewebe vom spitz zulaufenden Ende der Hülse seitlich verdrängt und um die Konnektion herumgeführt werden kann.

Vorteilhaft verringert sich der Außendurchmesser der Hülse am vom Träger wegweisenden Ende auf einen Durchmesser, welcher etwa gleich dem Außendurchmesser der Kanüle ist, so dass die Hülse an ihrem Außenbereich an dem Katheter anliegt. Jedoch kann der kleinste Außendurchmesser der Hülse auch so ausgebildet sein, dass die Hülse eine gewisse Distanz von zum Beispiel 0,1 bis 2 mm von der Katheteroberseite vorsteht, wobei dann vorteilhaft das Hülsenende so ausgestaltet ist, dass keine scharfen Kanten vorstehen, welche sich bei einem Verschieben der Konnektion in Gewebe wie Schneidkanten verhalten würden. So kann die Hülse z. B. am Ende leicht abgerundet sein.

Besonders vorteilhaft ist an mindestens einem Ende der Klemmhülse ein Innengewinde vorgesehen, welches mit dem Außengewinde eines Trägers verschraubt werden kann. Bevorzugt ist eine Schnapp- oder Rastverbindung vorgesehen, zum Beispiel in Gestalt mehrerer federnder Schnapphaken, welche in ein entsprechendes Verbindungselement auf dem Träger, wie zum Beispiel eine umlaufende Rille eingreifen können, um eine auf den Träger aufgebrachte Klemmhülse möglichst sicher mit dem Träger verbinden zu können.

Dabei können die beiden Enden des Trägers, an welchem die Klemmhülsen zur Befestigung der Katheterenden aufgebracht werden, in etwa identisch oder auch mit unterschiedlichen Merkmalen, wie zum Beispiel verschiedenen Gewinden oder Steck- bzw. Rastverbindungen ausgestattet werden, um eine eindeutige Zuordnung einer bestimmten Klemmhülse zu einem bestimmten Ende des Trägers sicher zu stellen.

Vorteilhaft sind der Träger und die mindestens eine Klemmhülse so aufeinander abgestimmt, dass eine Press- oder Klemmverbindung zum Befestigen eines Katheterendes zwischen der mindestens einen Klemmhülse und dem Träger hergestellt werden kann, welche zuverlässig ein Lösen des Katheters von dem Träger verhindern kann.

Bevorzugt sind einzelne oder alle der oben beschriebenen Komponenten der Erfindung aus biokompatiblem und/oder formstabilem Material, wie zum Beispiel Polyurethan, Silikon, Titan oder anderen geeigneten Stoffen, welche bezüglich der Gewebeverträglichkeit unbedenklich sind.

Nach einem weiteren Aspekt bezieht sich die vorliegende Erfindung auf ein System mit einer Katheterkonnektion und einem Portkörper, an welchem vorteilhaft bereits vor dem Einsetzen in Gewebe ein Katheterstück fest angebracht ist. Somit muss nach dem Einsetzen des Portkörpers lediglich das vom Portkörper abgehende Katheterstück mit dem bereits in das Gewebe eingesetzten Katheterstück mit der erfindungsgemäßen Katheterkonnektion verbunden werden, wobei bei geeigneter Länge der zu konnektierenden Katheterenden die Konnektion nicht in unmittelbarer Nähe bei einem geöffneten Gewebe erfolgen kann. Aufgrund der konischen Ausbildung der Hülsen kann die Katheterkonnektion leicht im Gewebe verschoben werden.

Die Erfindung wird nachfolgend anhand eines bevorzugten Ausführungsbeispieles beschrieben werden. Es zeigt:
- Figur 1: eine erfindungsgemäße Katheterkonnektion in perspektivischer Ansicht;
- Figur 2: eine Schnittansicht der in Figur 1 gezeigten Katheterkonnektion;
- Figur 3 a: einen Träger; und
- Figur 3b: eine Explosionsansicht einer erfindungsgemäßen Katheterkonnektion mit einem weiteren Träger und zwei Klemmhülsen.

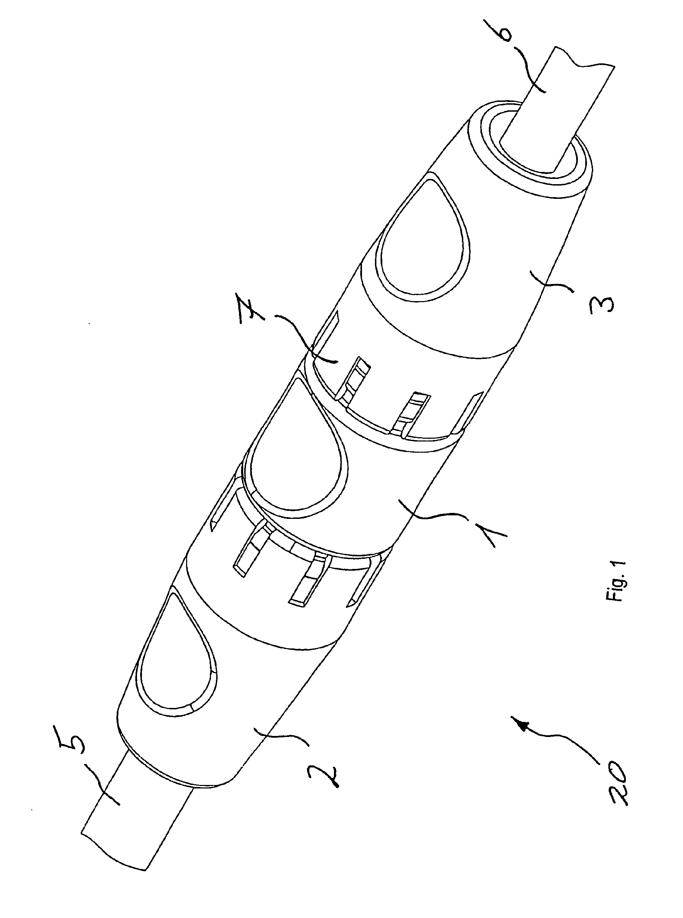

Figur 1 zeigt eine erfindungsgemäße Katheterkonnektion in perspektivischer Darstellung, wobei ein distales Katheterende 5 mit einem proximalen Katheterende 6 durch die Katheterkonnektion 20 verbunden ist.

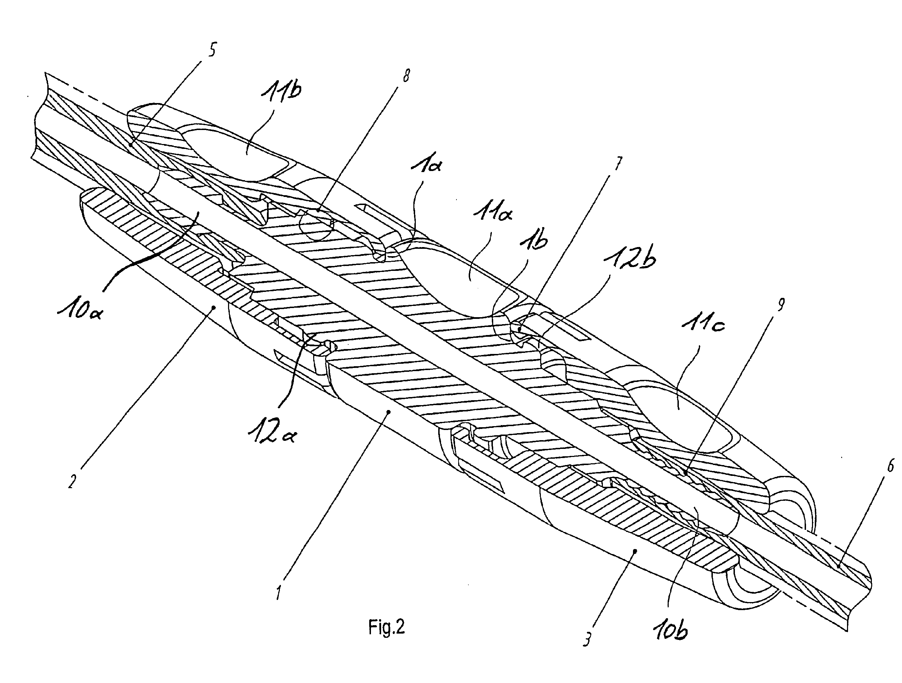

Figur 2 zeigt die Katheterkonnektion von Figur 1 in einer Teilschnittansicht, wobei erkannt werden kann, dass das distale Katheterende 5 auf das distale Ansetzrohr 10a aufgesetzt ist, dessen Innendurchmesser etwa gleich groß wie der Innendurchmesser des Katheters 5 ist. Das distale Ansetzrohr 10a des Trägers 1 ist in Richtung auf das distale Ende des Trägers 1 konisch zulaufend ausgebildet, um das Ansetzen des distalen Katheters 5 zu vereinfachen. In Richtung auf das proximale Ende weitet sich das distale Ansetzrohr 10a leicht auf und hat einen etwas größeren Außendurchmesser als am proximalen Ende, um durch den aufgeweiteten Bereich des distalen Ansetzrohres 10a ein gutes Festklemmen des distalen Katheters 5 zu ermöglichen.

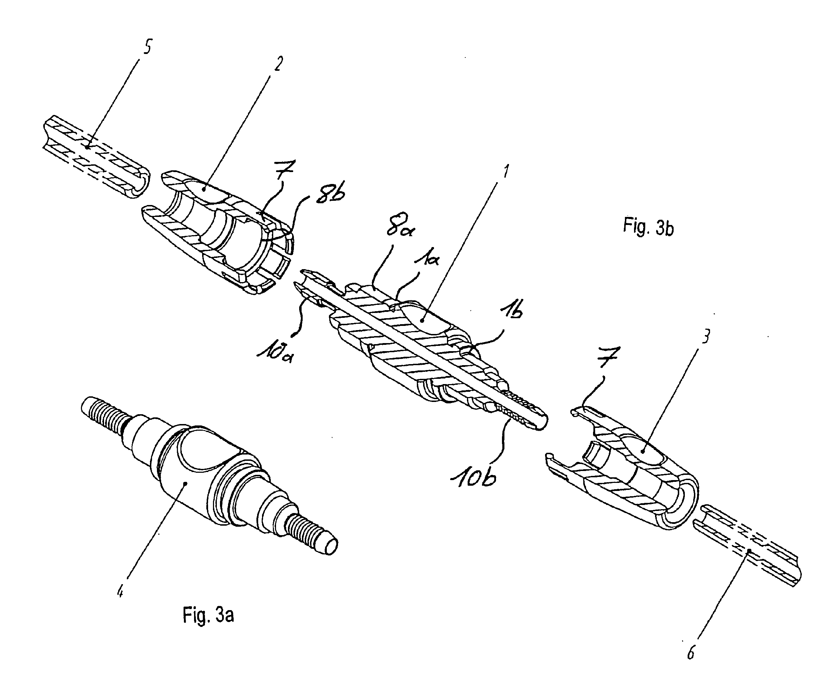

Vor dem Ansetzen des distalen Katheterendes 5 an den Träger 1 wird die distale Klemmhülse 2 auf den Katheter 5 aufgeschoben. Ist der Katheter 5 auf das Ansetzrohr 10a aufgeschoben worden, so kann die Klemmhülse 2 entlang des Katheters 5 in Richtung des Trägers 1 verschoben werden. In dem mit 8 bezeichneten um den Träger 1 umlaufenden Bereich greift ein in Figur 3b gezeigtes Innengewinde 8b der Klemmhülse 2 in ein Außengewinde 8a des Trägers 1 ein, wodurch die Klemmhülse 2 auf den Träger 1 aufgeschraubt werden kann, bis die an dem dem Träger 1 zugewandten Ende der Klemmhülse 2 vorgesehenen Schnapphaken 7 in eine Halterille 1a des Trägers 1 eingreifen können, um eine Rast- oder Federsicherung der Klemmhülse 2 an dem Träger 1 herzustellen, wodurch die Gewindeverbindung 8 entlastet werden kann und ein unbeabsichtigtes Lösen der Klemmhülse 2 von dem Träger 1 durch zum Beispiel versehentliches Aufdrehen des Gewindeverschlusses 8 verhindert werden kann. Die Rastverbindung wird durch mehrere z. B. acht um den Umfang der Klemmhülse 2 herum vorgesehene Schnapphaken 7 realisiert, welche an länglichen vorstehenden Teilstücken der Klemmhülse 2 so ausgebildet sind, dass die Schnapphaken 7 in radialer Richtung der Klemmhülse 2 federn können, um so beim Aufdrehen der Klemmhülse 2 auf die Trägerhülse 1 den vor der Halterille 1a vorstehenden ringförmigen Vorsprung zu überfahren und anschließend in die Halterille 1a einzugreifen.

Am proximalen Ende des Trägers 1 ist ein proximales Ansetzrohr 10b vorgesehen, auf welches das proximale Katheterende 6 aufgeschoben werden kann. Das proximale Ansetzrohr 10b weist an seiner Oberseite eine gerippte Struktur auf, welche durch mehrere hintereinander liegende umlaufende Aufweitungen am Ansetzrohr 10b ausgebildet sind, um hierdurch das Ansetzen und Festklemmen des proximalen Katheterendes 6 auf dem proximalen Ansetzrohr 10b zu vereinfachen. Die proximale Klemmhülse 3 wird ebenso wie die oben beschriebene distale Klemmhülse 2 zunächst auf den proximalen Katheter 6 aufgeschoben. Danach wird der Katheter 6 an das Ansetzrohr 10b angesetzt und mit der darüber geschobenen Klemmhülse 3 durch Verschrauben und Verrasten der Klemmhülse 3 an der Halterille 1b mittels der Schnapphaken 7 zwischen dem Ansetzrohr 10b und der Klemmhülse 3 festgeklemmt.

Wie aus Figur 3a ersichtlich, kann die in Figur 3b gezeigte Trägerhülse 1 in einer alternativen Ausführungsform auch andere Ausgestaltungen der Ansetzrohre 10a und 10b aufweisen, welche in dem gezeigten Beispiel beide eine gerillte Oberflächenstruktur aufweisen. Allgemein kann jede Kombination von gleichen oder unterschiedlichen Ausformungen der distalen und proximalen Ansetzrohre 10a und 10b verwendet werden, welche zum Beispiel eine glatte, geriffelte oder strukturierte Oberfläche aufweisen können.

Zur Vereinfachung der Handhabung sind auf der Trägerhülse 1 und den Klemmhülsen 2 und 3 Haltebereiche 11a, 11b und 11c ausgebildet, welche das Halten und Verdrehen der Trägerhülse 1 und der Klemmhülsen 2 und 3 vereinfachen.

Zur Diskonnektion der Katheter 5 und 6 wird der oben beschriebene Vorgang in umgekehrter Reihenfolge durchgeführt, d.h. zunächst wird die Rast- oder Schnappverbindung der Schnapphaken 7 in der entsprechenden Halterille 1a oder 1b gelöst, indem ein Druck auf die federnden Elemente 7 in dem Bereich ausgeübt wird, welcher dem Hauptkörper der jeweiligen Klemmhülse 2 oder 3 zugewandt ist, d.h. im Bereich der der Klemmhülse 2 oder 3 zugewandten Seite des Schnapphakens 7, wodurch das vordere Ende des jeweiligen Schnapphakens 7 durch eine Hebelkraft, welche über die als Auflagepunkt für den Hebel wirkende Erhebung 12a bzw. 12b übertragen wird, nach außen aus der jeweiligen Halterille 1a bzw. 1b gehoben wird, um so die Schnapp- oder Rastverbindung freizugeben. Anschließend kann die Klemmhülse 2, 3 von dem Träger 1 abgeschraubt und entlang des Katheters 5, 6 zurückgeschoben werden, um die Klemmverbindung des entsprechenden Katheters 5, 6 auf dem Ansetzrohr 10a, 10b zu lösen. Danach kann der Katheter 5, 6 vom Ansetzrohr 10a, 10b abgezogen und die Klemmhülse 2, 3 von dem Katheter 5, 6 entfernt werden. Die durch die erfindungsgemäße Katheterkonnektion ausgebildete Katheterverbindung ist somit einfach reversibel konnektier- und lösbar.

In den Figuren nicht gezeigt ist ein perkutanes Portsystem, welches sich bevorzugt in der Nähe der erfindungsgemäßen Katheterverbindung 20 befindet, zum Beispiel ca. 5 cm versetzt davon. Dabei kann die Katheterkonnektion 20 in derselben subkutanen Hauttasche wie der Portkörper vorgesehen werden, wobei der Portkörper zum Beispiel schon vor dem Einsetzen fest mit dem proximalen Katheter 6 verbunden sein kann und nach dem Einsetzen nicht mehr mit dem proximalen Katheter 6 verbunden werden muss.

Über den Portkörper können verschiedene Infusionslösungen, wie zum Beispiel eine Nutritionslösung, diverse Medikamentenlösungen, Cytostatika oder diverse Spüllösungen über den proximalen Katheter 6 durch die erfindungsgemäße Katheterkonnektion 20 hindurch zu dem distalen Katheter 5 in den gewünschten Körperbereich eingebracht werden. Wird der Innendurchmesser des Trägers etwa so groß ausgebildet wie der Innendurchmesser der zu konnektierenden Katheter, so kann aufgrund des im Wesentlichen gleichbleibenden Querschnitts ein laminarer Fluss eines Mediums gewährleistet werden.

Die erfindungsgemäße Katheterkonnektion 20 kann demzufolge einfach und im Wesentlichen ohne nennenswerte Zugbelastungen der distalen oder proximalen Katheter 5, 6 angebracht werden und kann aufgrund der konischen Ausformung im Bereich der Klemmhülsen 2 und 3 relativ leicht durch umliegendes Gewebe bei geringer Gefahr der Verletzung des Gewebes bewegt werden, falls die Katheterkonnektion 20 aus dem Gewebe herausgezogen oder an einen bestimmten Bereich des Gewebes geschoben werden soll.

## Patentansprüche

1. Katheterkonnektion (20) mit einem Träger (1, 4) und mindestens zwei Hülsen (2, 3), wobei der Träger (1, 4) zum Befestigen mindestens zweier Katheterenden (5, 6) mindestens zwei Ansetzelemente (10a, 10b) aufweist, an welche die Katheterenden (5, 6) angesetzt werden können und mindestens zwei Verbindungsstrukturen (1a, 1b; 8a) aufweist, an welchen jeweils eine Hülse (2, 3) zum Befestigen der mindestens zwei Katheterenden (5, 6) befestigt werden kann und wobei die mindestens zwei Hülsen (2, 3) auf den Katheter aufgeschoben werden können.

2. Katheterkonnektion nach Anspruch 1, wobei drei oder vier bis zehn Ansetzelemente (10a, 10b) an dem Träger (1) vorgesehen sind.

3. Katheterkonnektion nach Anspruch 1 oder 2, wobei die Verbindungsstruktur ein Außengewinde oder Gewindeabschnitt (8a) der Trägerhülse (1) ist.

4. Katheterkonnektion nach einem der vorhergehenden Ansprüche, wobei die Verbindungsstruktur eine Oberflächenstruktur für eine Schnapp- oder Rastverbindung, insbesondere eine Halterille (1a, 1b) ist.

5. Katheterkonnektion nach einem der vorhergehenden Ansprüche, wobei das mindestens eine Ansetzelement (10a, 10b) etwa röhrenförmig ausgebildet ist und/oder an seinem Außenumfang eine glatte, geriffelte oder strukturierte Oberfläche aufweist.

6. Katheterkonnektion nach einem der vorhergehenden Ansprüche, wobei die Hülsen (2, 3) einen konisch zulaufenden Bereich aufweisen.

7. Katheterkonnektion nach Anspruch 6, wobei der konisch zulaufende Bereich bis etwa auf den Außenumfang eines Katheters zuläuft.

8. Katheterkonnektion nach einem der vorhergehenden Ansprüche, wobei die Hülse (2,3) ein Innengewinde (8b) aufweist.

9. Katheterkonnektion nach einem der vorhergehenden Ansprüche, wobei mindestens ein Rast- oder Schnappverbindungselement (7) an der Hülse (2,3) vorgesehen ist.

10. Katheterkonnektion nach Anspruch 9, wobei das mindestens eine Rast- oder Schnappverbindungselement (7) federnd an der Hülse (2,3) vorgesehen ist.

11. Katheterkonnektion nach einem der vorhergehenden Ansprüche, wobei der Träger (1, 4) und/oder die Klemmhülsen (2, 3) zumindest zum Teil aus biokompatiblem Material, insbesondere Polyurethan, Silikon oder Titan hergestellt sind.

12. System mit einer Katheterkonnektion (20) nach einem der vorhergehenden Ansprüche und einem Portkörper.

13. Verfahren zum Befestigen mindestens zweier Katheterenden (5, 6) mit einer Katherkonnektion gemäß einem der Ansprüche 1 bis 11, wobei eine erste Hülse (2) auf ein erstes Katheterende (5) und eine zweite Hülse (3) auf ein zweites Katheterende (6) aufgeschoben wird, das erste Katheterende (5) an ein erstes Ansetzelement (10a) aufgeschoben und das zweite Katheterende (6) auf ein zweites Ansetzelement (10b) aufgeschoben wird und wobei die Hülsen (2, 3) über die angesetzten Katheterenden (5, 6) geschoben werden und die Katheterenden (5, 6) durch Einklemmen der Katheterenden (5, 6) zwischen dem Träger (1, 4) und der jeweiligen Hülse (2, 3) befestigt werden.

## Claims

1. A catheter connection (20) comprising a support (1, 4) and at least two sleeves (2, 3), wherein the support (1, 4) for fastening at least two catheter ends (5, 6) comprises at least two joining elements (10a, 10b) onto which the catheter ends (5, 6) can be placed, and at least two connecting structures (1a, 1b; 8a), to each of which a sleeve (2, 3) for fastening the at least two catheter ends (5, 6) can be fastened, and wherein the at least two sleeves (2, 3) can be slid onto a catheter.

2. The catheter connection according to claim 1, wherein three or four to ten joining elements (10a, 10b) are provided on the support (1).

3. The catheter connection according to claim 1 or 2, wherein the connecting structure is an outer thread or threaded section (8a) of the support sleeve (1).

4. The catheter connection according to any one of the preceding claims, wherein the connecting structure is a surface structure for a latching or locking connection, in particular a holding groove (1a, 1b).

5. The catheter connection according to any one of the preceding claims, wherein the at least one joining element (10a, 10b) is formed to be roughly tubular and/or comprises a smooth, ribbed or structured surface on its outer circumference.

6. The catheter connection according to any one of the preceding claims, wherein the sleeves (2, 3) comprise a conically tapering area.

7. The catheter connection according to claim 6, wherein the conically tapering area tapers up to approximately the outer circumference of a catheter.

8. The catheter connection according to any one of the preceding claims, wherein the sleeve (2, 3) comprises an inner thread (8b).

9. The catheter connection according to any one of the preceding claims, wherein at least one locking or latching connection element (7) is provided on the sleeve (2, 3).

10. The catheter connection according to claim 9, wherein the at least one locking or latching connection element (7) is provided elastically on the sleeve (2, 3).

11. The catheter connection according to any one of the preceding claims, wherein the support (1, 4) and/or the clamping sleeves (2, 3) are produced at least partially from bio-compatible material, in particular polyurethane, silicone or titanium.

12. A system comprising a catheter connection (20) according to any one of the preceding claims and a port body.

13. A method for fastening at least two catheter ends (5, 6) using a catheter connection in accordance with any one of claims 1 to 11, wherein: a first sleeve (2) is slid onto a first catheter end (5) and a second sleeve (3) is slid onto a second catheter end (6); the first catheter end (5) is slid onto a first joining element (10a) and the second catheter end (6) is slid onto a second joining element (10b); and the sleeves (2, 3) are slid over the joined catheter ends (5, 6) and the catheter ends (5, 6) are fastened by pinching the catheter ends (5, 6) between the support (1, 4) and the respective sleeve (2, 3).

## Revendications

1. Raccord de cathéter (20) avec un support (1, 4) et au moins deux douilles (2, 3), le support (1, 4) présentant au moins deux éléments d'insertion (10a, 10b) pour la fixation d'au moins deux extrémités de cathéter (5, 6), dans lesquels les extrémités de cathéter (5, 6) peuvent être insérées, et au moins deux structures d'assemblage (1a, 1b; 8a), auxquelles respectivement une douille (2, 3) de fixation des au moins deux extrémités de cathéter (5, 6) peut être fixée, et les au moins deux douilles (2, 3) pouvant être glissées sur le cathéter.

2. Raccord de cathéter suivant la revendication 1, trois ou quatre à dix éléments d'insertion (10a, 10b) étant prévus sur le support (1).

3. Raccord de cathéter suivant la revendication 1 ou 2, la structure d'assemblage étant un filet extérieur ou une partie filetée (8a) de la douille support (1).

4. Raccord de cathéter suivant l'une des revendications précédentes, la structure d'assemblage étant une structure de surface pour un assemblage à encliquetage ou à verrouillage, en particulier une rainure de retenue (1a, 1b).

5. Raccord de cathéter suivant l'une des revendications précédentes, l'au moins un élément d'insertion (10a, 10b) étant de forme environ tubulaire et/ou présentant à sa périphérie extérieure une surface lisse, cannelée ou structurée.

6. Raccord de cathéter suivant l'une des revendications précédentes, les douilles (2, 3) présentant une zone à rétrécissement conique.

7. Raccord de cathéter suivant la revendication 6, la zone à rétrécissement conique se rétrécissant jusqu'à environ la périphérie extérieure du cathéter.

8. Raccord de cathéter suivant l'une des revendications précédentes, la douille (2, 3) présentant un filet intérieur (8b).

9. Raccord de cathéter suivant l'une des revendications précédentes, au moins un élément d'assemblage à verrouillage ou encliquetage (7) étant prévu sur la douille (2,3).

10. Raccord de cathéter suivant la revendication 9, l'au moins un élément d'assemblage à verrouillage ou encliquetage (7) étant prévu de façon à s'appuyer élastiquement sur la douille (2,3).

11. Raccord de cathéter suivant l'une des revendications précédentes, le support (1, 4) et/ou les douilles de serrage (2, 3) étant fabriquées au moins en partie dans un matériau biocompatible, en particulier en polyuréthanne, silicone ou titane.

12. Système avec un raccord de cathéter (20) suivant l'une des revendications précédentes et un corps faisant port.

13. Procédé de fixation d'au moins deux extrémités de cathéter (5, 6) avec un raccord de cathéter suivant l'une des revendications 1 à 11, une première douille (2) étant glissée sur une première extrémité de cathéter (5) et une deuxième douille (3) étant glissée sur une deuxième extrémité de cathéter (6), la première extrémité de cathéter (5) étant glissée sur un premier élément d'insertion (10a) et la deuxième extrémité de cathéter (6) étant glissée sur un deuxième élément d'insertion (10b) et les douilles (2, 3) étant glissées sur les extrémités de cathéter insérées (5, 6) et les extrémités de cathéter (5, 6) étant fixées par serrage entre le support (1, 4) et la douille respective (2, 3).
